# EUROPEAN PATENT SPECIFICATION

(11) **EP 3 481 836 B1**
(45) Date of publication and mention of the grant of the patent: **09.12.2020**
(21) Application number: 17746547.3
(22) Date of filing: 05.07.2017
(51) Int. Cl.: C07D 513/04, A61K 31/519, A61P 25/00, A61P 29/00, A61P 35/00, A61P 17/00, A61K 45/06

(54) **NOVEL THIAZOLO[5,4-D]PYRIMIDINE DERIVATIVES AS INVERSE AGONISTS OF A2A ADENOSINE RECEPTORS**
NEUARTIGE THIAZOLO[5,4-D]PYRIMIDIN-DERIVATE ALS INVERSE AGONISTEN VON A2A-ADENOSINE-REZEPTOREN
NOUVEAUX DÉRIVÉS DE THIAZOLO [5,4-D]PYRIMIDINE EN TANT QU'AGONISTES INVERSES DES RÉCEPTEURS DE L'ADÉNOSINE A2A

(30) Priority: 07.07.2016 IT 201600070952
(43) Date of publication of application: 15.05.2019
(73) Proprietor: Universita' Degli Studi di Firenze, 50121 Firenze (IT); Università Degli Studi Di Ferrara, 44121 Ferrara (IT)
(72) Inventor: VARANO, Flavia, 50036 Vaglia (Firenze) (IT); COLOTTA, Vittoria, 50135 Firenze (IT); CATARZI, Daniela, 50145 Firenze (IT); VARANI, Katia, 44123 Ferrara (IT); BOREA, Pier Andrea, 40121 Ferrara (IT); VINCENZI, Fabrizio, 44121 Ferrara (IT)
(74) Representative: Brazzini, Silvia
(86) International application number: PCT/IB2017/054049
(87) International publication number: WO 2018/007957

(56) References cited:
- WO-A1-2005/028484
- FLAVIA VARANO ET AL: "Design, Synthesis, and Pharmacological Characterization of 2-(2-Furanyl)thiazolo[5,4- d ]pyrimidine-5,7-diamine Derivatives: New Highly Potent A 2A Adenosine Receptor Inverse Agonists with Antinociceptive Activity", JOURNAL OF MEDICINAL CHEMISTRY, vol. 59, no. 23, 8 December 2016 (2016-12-08), pages 10564-10576, XP055337740, ISSN: 0022-2623, DOI: 10.1021/acs.jmedchem.6b01068

## Description

### Field of the invention

The present invention generally relates to the pharmaceutical field, and more precisely relates to novel thiazolo[5,4-d]pyrimidine derivatives of formula (I) reported below, which are inverse agonists of adenosine A_{2A} receptor, useful for the treatment neurological diseases, pain, cancer, dermal fibrosis and scarring.

### State of the Art

Adenosine, a well-known purine nucleoside, acts as an endogenous modulator in the human body both in the central nervous system and in the peripheral nervous system, by interacting with four receptors coupled to G protein (GPCRs) identified as adenosine receptors (ARs) A₁, A_{2A}, A_{2B}, and A₃ that are expressed ubiquitously (Fredholm BB et al., Pharmacol. Rev. 2011; 63: 1-34).

Important advances have been made in understanding the role of adenosine receptors under physiologic conditions and in a variety of pathologies through the potential use of agonists, antagonists and inverse agonists.

It has been reported in the literature that in the central nervous system there is a co-expression of A_{2A} adenosine receptors with dopamine D₂ receptors in GABAergic striatopallidal neurons where adenosine and dopamine exert opposite effects in regulating locomotor activity. Epidemiological studies have found a strong association between caffeine consumption, a non-selective A_{2A} receptor antagonist and a reduced risk of developing Parkinson's disease. Furthermore, it has been observed that treatment with an A_{2A} receptor antagonist could potentiate the effect of L-DOPA, a precursor of dopamine, and reduce various characteristic motor symptoms of Parkinson's disease such as tremor or dyskinesia (Armentero MT et Al., Pharmacol. Ther., 2011; 132: 280-299).

Recent studies have shown that both caffeine and A_{2A} receptor antagonists prevent the accumulation of β-amyloid peptide (Aβ) in the brain blood vessels and in their proximity, accumulation which, if not treated, could lead to cognitive deficits. In an *in vivo* mouse model of Alzheimer's disease, chronic consumption of caffeine regresses cognitive deficits and decreases the Aβ levels in the brain. In addition, caffeine favours the survival of neurons and slows down the process of neurodegeneration in the streaked body and/or the cerebral cortex, and this can contribute to its beneficial effects against Alzheimer's disease.

The important role of glutamatergic neurotransmission in Huntington's disease and the positive effects of A_{2A} receptor antagonists such as SCH58261 or of inverse antagonists / agonists such as ZM 241385 in animal models of Huntington's disease are well known. The potential effect of neuroprotection of the A_{2A} receptors on epileptic states is based on the effect of ZM 241385, which shows a good anticonvulsant profile with few side effects. The A_{2A} receptors blockade contributes to a significant protection in the central nervous system after spinal cord injury by reducing excessive release of neurotransmitters caused by high levels of intracellular calcium ions, which can lead to neuronal death following increased excitotoxicity.

*In vivo* studies have shown the involvement of A_{2A} receptors in the nociceptive response. Knockout mice, in which A_{2A} receptor genes were suppressed, were less susceptible to nociceptive stimulation probably due to the lack of A_{2A} pro-nociceptive receptors on the sensory nerves. In addition, the well-known reverse A_{2A} antagonist / agonist ZM 241385, injected into the back paw, reduced mechanical hyperalgesia following carrageenan injection into mice. Double blind studies in humans compared the effect in treating acute pain of a single dose of analgesic and caffeine with the same dose of analgesic alone. The addition of caffeine to a standard dose of commonly used analgesics resulted in a significant increase of the analgesic effect of the compound in the majority of participants in the study (Derry C. J. et al., Cochrane Database Syst. Rev. 2014; 12: CD009281).

Significant evidences show a protective role of A_{2A} antagonists in striatal and nigral neurons through the prevention of glutamate-induced neuronal death, thus reducing cortical damage in different ischemic stroke models. The selective antagonist of A_{2A} SCH58261 reduced brain ischemic damage in a model of cerebral focal ischemia in rat.

The application of the selective A_{2A} reverse antagonist / agonist, ZM 241385, reduces the scar's size and increases the traction shear strength due to an improved collagen structure. In addition, treatment with ZM 241385 has been shown to reduce the number of myofibroblasts and angiogenesis in the scar, but did not affect macrophage infiltration in the scar. It has been shown that adenosine is involved in the pathogenesis of dermal fibrosis and in the development of fibrosis in murine models of scleroderma and cirrhosis, suggesting a potential role for A_{2A} inverse antagonists / agonists in the treatment and prevention of fibrosis (Chan & Cronstein, Mod. Rheumatol. 2010; 20: 114-122). A_{2A} receptors are increased in fibroblasts in cases of scleroderma and produce significant fibrogenic effects, suggesting that A_{2A} antagonists may be useful in the treatment of dermal fibrosis. Mice treated with ZM 241385 are protected against the development of bleomycin-induced dermal fibrosis through the regulation mediated by the A_{2A} receptor of the recruitment of fibrocytes towards the dermis.

It has also been noted that A_{2A} receptors activation significantly increases the proliferation of tumour cell lines and favours tumour angiogenesis, due to the high level of expression of the receptor associated with endothelial cells. Preclinical studies indicate that adenosine in the tumour microenvironment strongly weakens T cell anticancer response and that ZM 241385 may increase the antitumor effect of these cells. Therefore, blocking adenosine-induced immunosuppression by inhibiting A_{2A} receptors with ZM 241385 may improve immunological cancer therapy, including antitumor vaccination.

To date, several pharmaceutical companies are involved in the organization of clinical studies with A_{2A} receptor antagonists, such as KW6002 or istradefylline of Kyowa Hakko Kirin Co; ST1535 of Sigma-Tau; Tozadenant (SYN115) from Biotie Therapies & UCB Pharma; V81444 and Vipadenant (V2006) of Vernalis-Biogen; PBF509 from Palobiofarma; and Preladenant (SCH420814) of Merck & Co. (Pretti D. et al., Med. Res. Rev., 35: 790-848).

In general, it is well known that agonist compounds activate receptors to produce the desired response and increase the proportion of activated receptors, while antagonists inhibit or anyway reduce receptor response due to the action of an agonist with different modes, depending on the fact that they compete with the agonist for the same binding site to the receptor (competitive antagonists) or they are linked to it on a different site (non-competitive antagonists). Compounds that are instead inverse agonists of a given receptor stabilize it in its inactive form and act in a similar manner to competitive antagonists that block the action of the receptor agonists. In addition, compounds that are inverse receptor agonists not only block the effects of agonists, as a classic antagonist would do, but they also inhibit the basal activity of the receptor (Kenakin T., Trends Pharmacol. Sci. 2014; 35: 434- 441).

As described above, the search for new ligands for the A_{2A} receptor of adenosine has been remarkably established and in the last few years a large number of A_{2A} adenosine receptor antagonists have been synthesized, with potentially therapeutic and pharmacological effects in various diseases. WO 2005028484 A1 discloses thiazolopyridyl compounds useful in particular for treating or preventing diseases based on adenosine A_{2A} receptor antagonistic activity.

The need to identify new compounds that not only block the effects of agonists, but that are inverse agonists of adenosine A_{2A} receptor, according to the above-clarified meaning, is still very much felt.

As a matter of fact, a compound that acts as an inverse agonist towards a receptor does not only have an inhibitory function of the endogenous agonist effect, but if a certain disease or pathological condition is deteriorated by the constituent activity of the receptor, only a compound that is an inverse agonist may be useful in attenuating this activity and consequently improving the pathological condition in question.

### Summary of the invention

Now the Applicants have synthesised novel thiazolo[5,4-d]pyrimidine derivatives of formula (I) illustrated below having a high affinity for the adenosine receptor A_{2A}; they proved to be excellent ligands for such receptor with high potency as inverse agonists as disclosed in details in the following.

It is therefore subject of the invention the compounds of general formula (I) 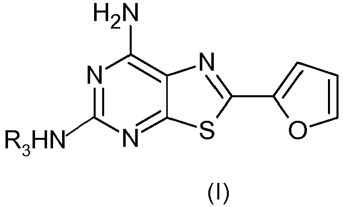 wherein R₃ is selected from the group consisting of hydrogen, alkyl optionally substituted, (CH₂)ₙaryl optionally substituted and (CH₂)ₙheteroaryl optionally substituted, wherein n is an integer ranging from 0 to 4,
and pharmaceutically acceptable salts, tautomers or enantiomers thereof.

Further subject of the invention are the compounds of general formula (I) defined above for the use as medicament, and in particular for the use in the therapeutic treatment of diseases or disorders responsive to the blockade of the adenosine A_{2A} receptor.

A process for the preparation of the compounds of general formula (I) defined above is a further subject of the invention.

A pharmaceutical composition comprising at least a compound of formula (I) in admixture with one or more pharmaceutically acceptable excipients and/or diluents is still a further subject of the invention.

Other important features of the compounds of formula (I), of the process for preparing them, of the pharmaceutical compositions comprising them and of the related medical use according to the invention are reported in the following detailed description.

### Brief description of the figures

The Figures from 1 to 6 here attached show some of the most significant results obtained in the experimental studies described in detail in the following in Example 6. In particular:

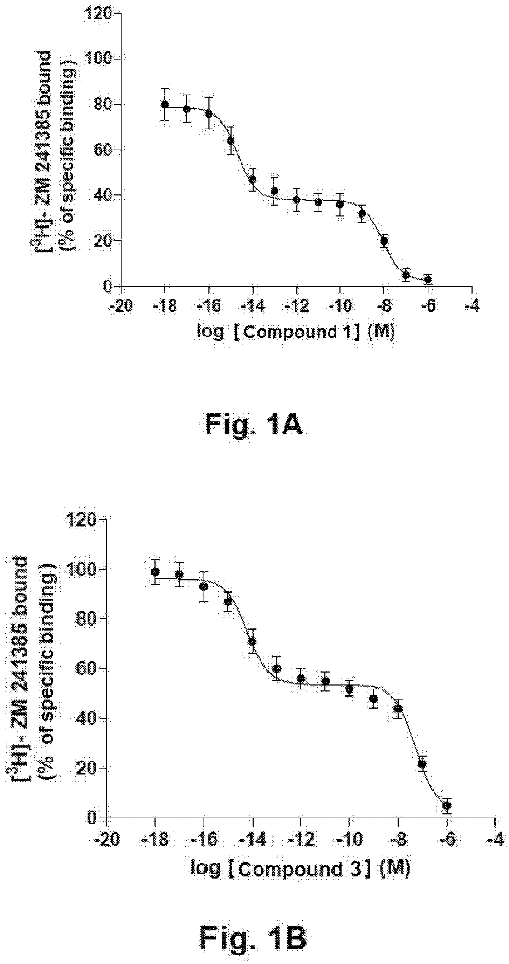

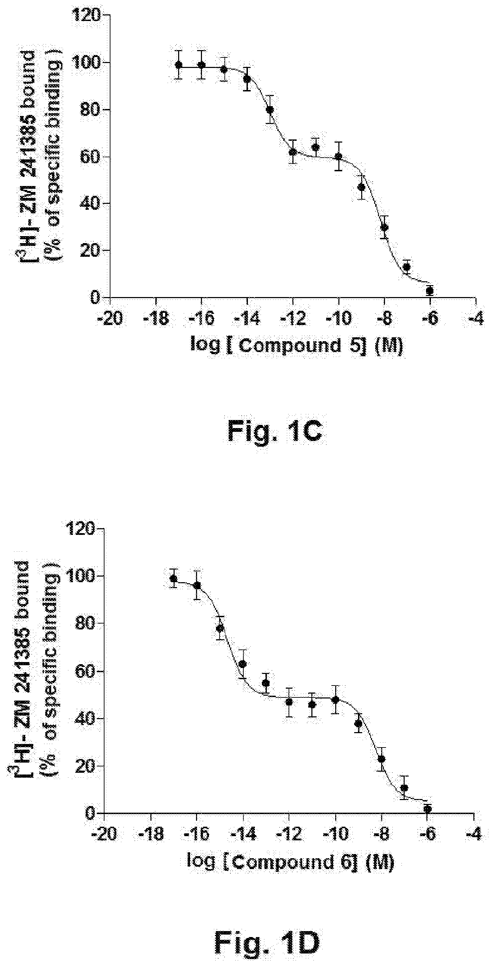

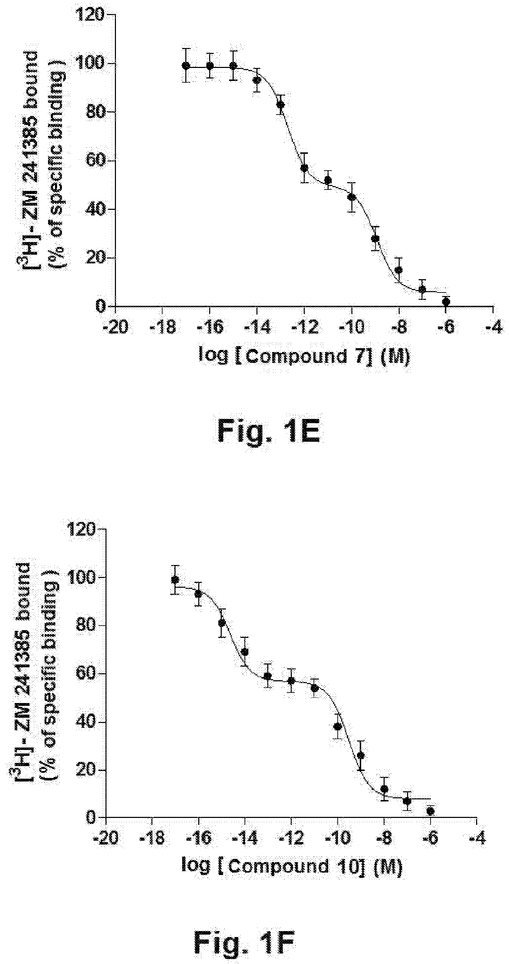

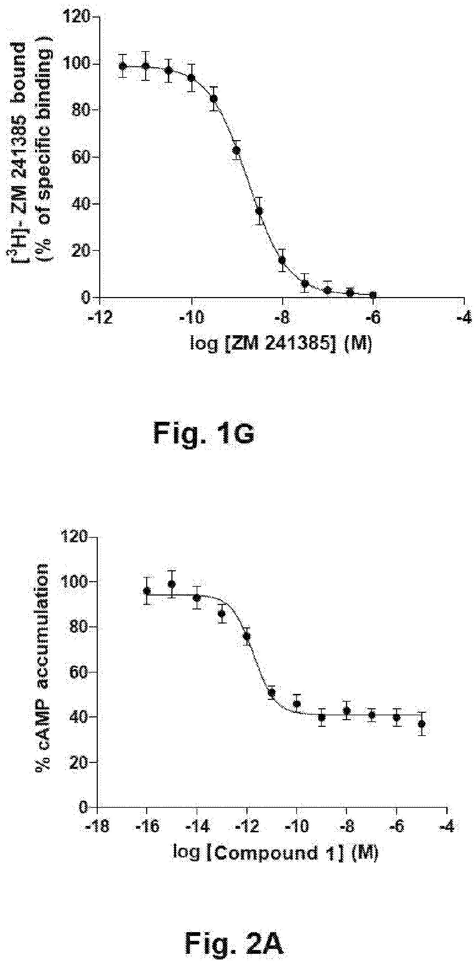

- Figures 1A, 1B, 1C, 1D, 1E, 1F and 1G represent the competition curves of [³H]-ZM 241385 at the human receptor A_{2A} of adenosine for compound 1 (Fig. 1A), compound 3 (Fig. 1B), compound 5 (Fig. 1C), compound 6 (Fig. 1D), compound 7 (Fig. 1E), and compound 10 (Fig. 1F) of the present invention, which show the presence of two binding sites while the reference compound ZM 241385 (Fig. 1G) is characterised by single-phase curve;

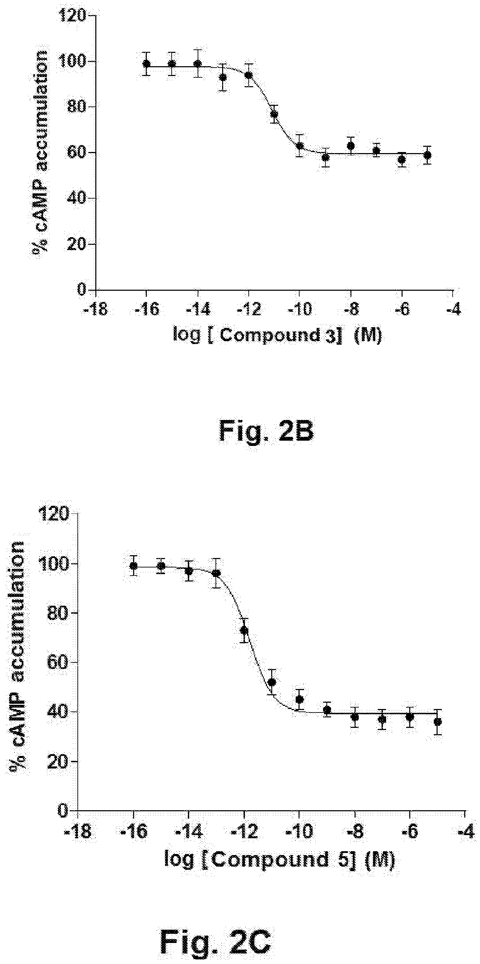

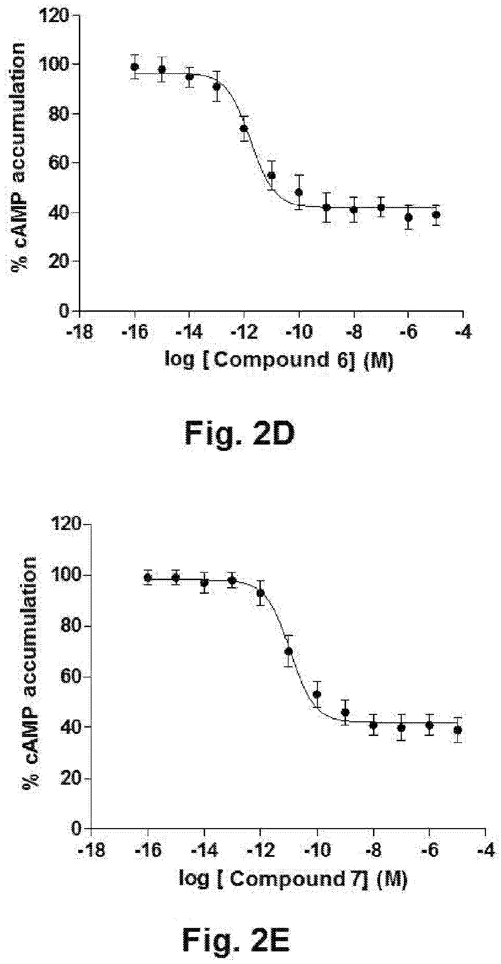

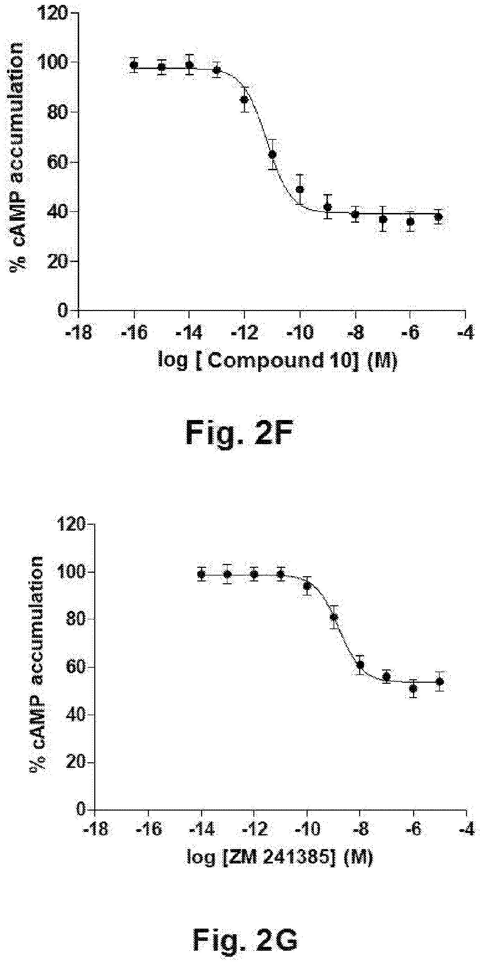

- Figures 2A, 2B, 2C, 2D, 2E, 2F and 2G show the inhibition curves of the cAMP levels in CHO cells transfected with the human receptor A_{2A} obtained for the compound 1 (Fig. 2A), for the compound 3 (Fig. 2B), for the compound 5 (Fig. 2C), for the compound 6 (Fig. 2D), for the compound 7 (Fig. 2E), and for the compound 10 (Fig. 2F) of the present invention, and for the reference compound ZM 241385 (Fig. 2G); in these figures the effects of the active compound are expressed as percentage with respect to the basal production of cAMP;

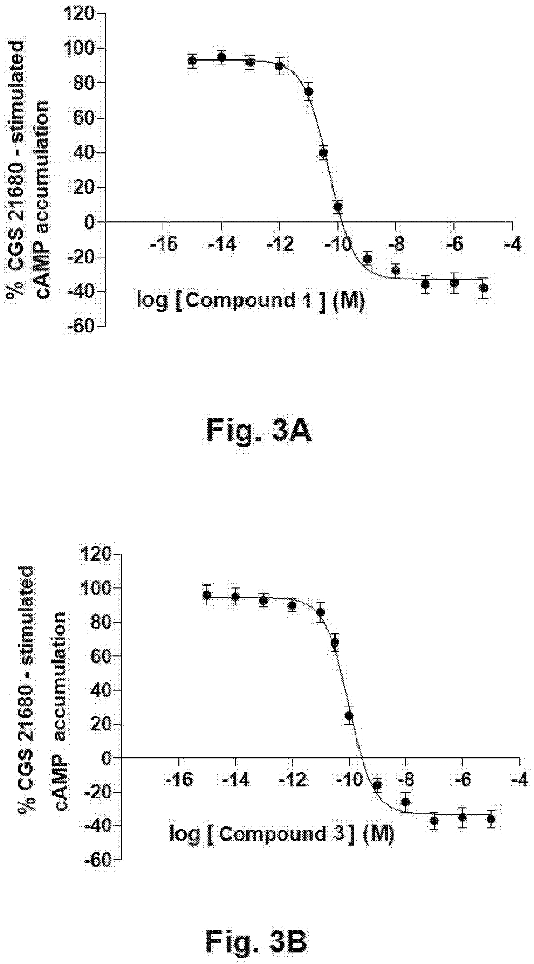

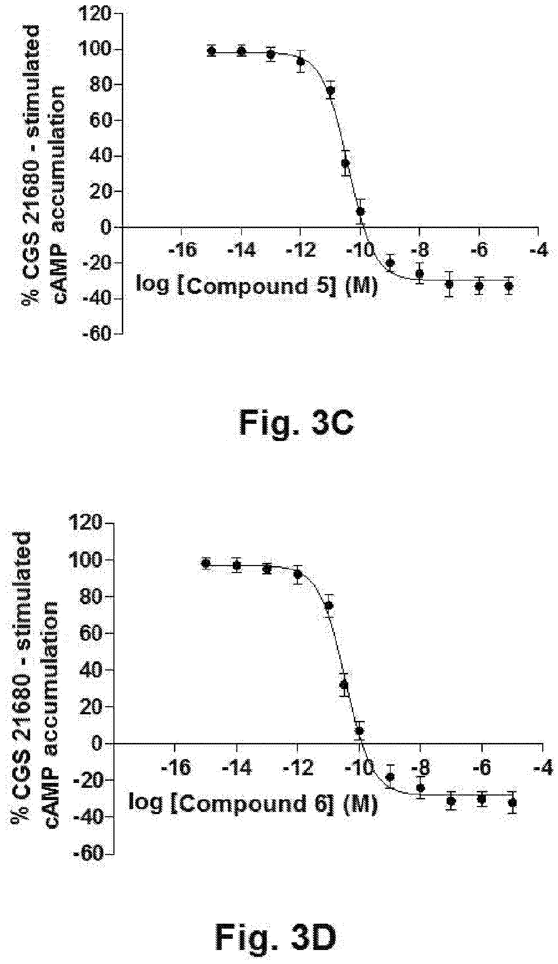

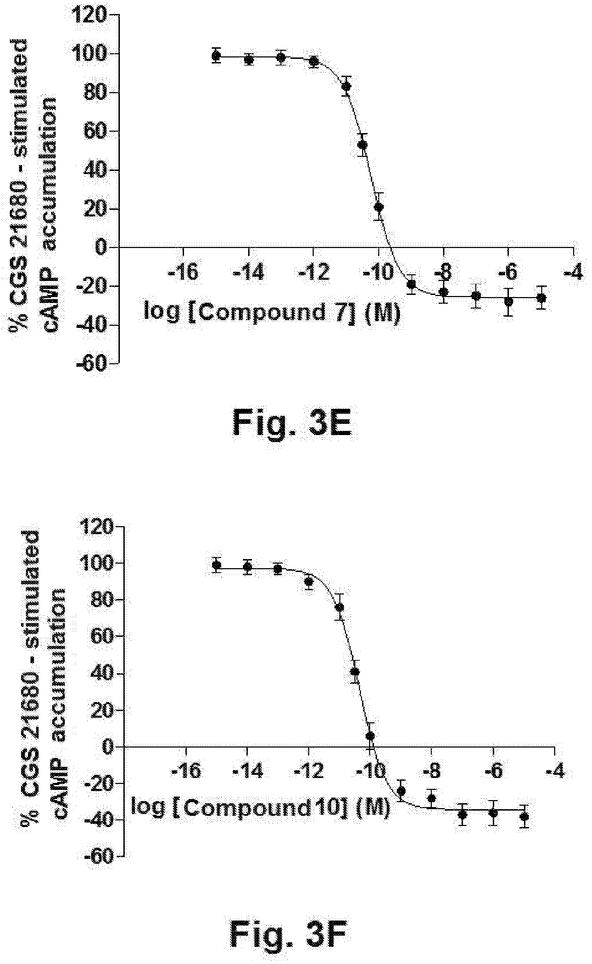

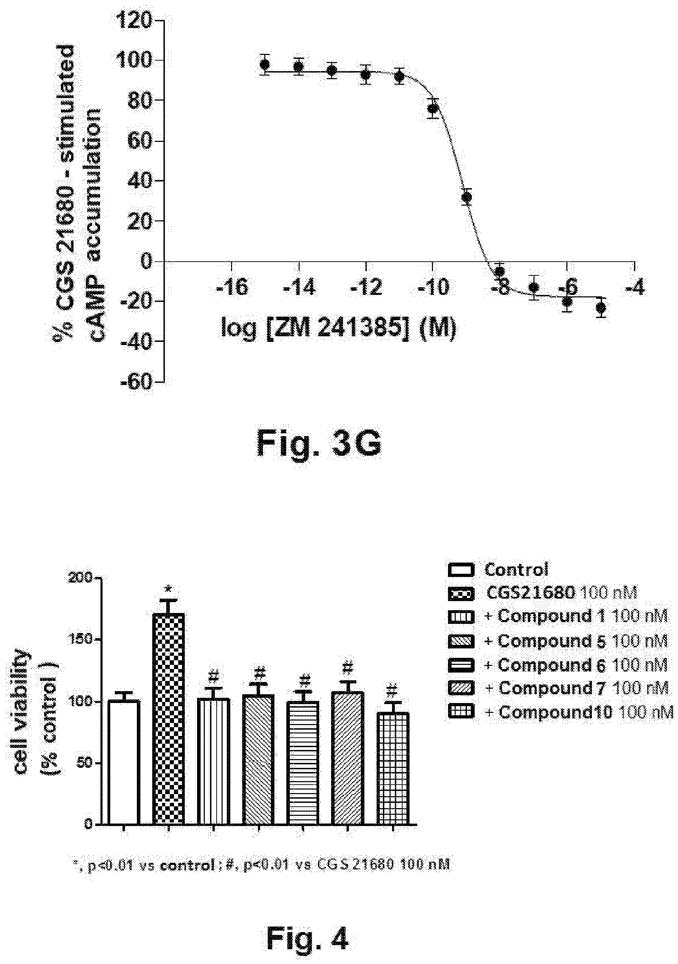

- Figures 3A, 3B, 3C, 3D, 3E, 3F and 3G show the inhibition curves of the cAMP levels in CHO cells transfected with the human receptor A_{2A} obtained for the compound 1 (Fig. 3A), for the compound 3 (Fig. 3B), for the compound 5 (Fig. 3C), for the compound 6 (Fig. 3D), for the compound 7 (Fig. 3E), and for the compound 10 (Fig. 3F) of the present invention, and for the reference compound ZM 241385 (Fig. 3G), wherein the effects of the compounds are expressed as percentage with respect to the production of cAMP in the presence of CGS 21680 (10 nM);
- Figure 4 shows, in the form of histograms, the percentages of cells viability with respect to a control for samples of breast cancer cells MRMT-1 treated with CGS 21680 and with the compounds 1, 5, 6, 7 and 10 of the present invention, according to what described in the experiments of cellular proliferation of the following Example 6;

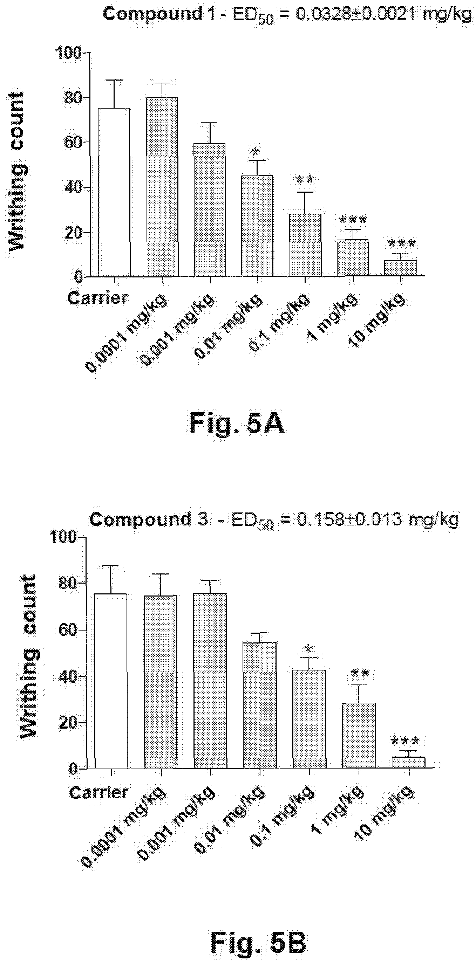

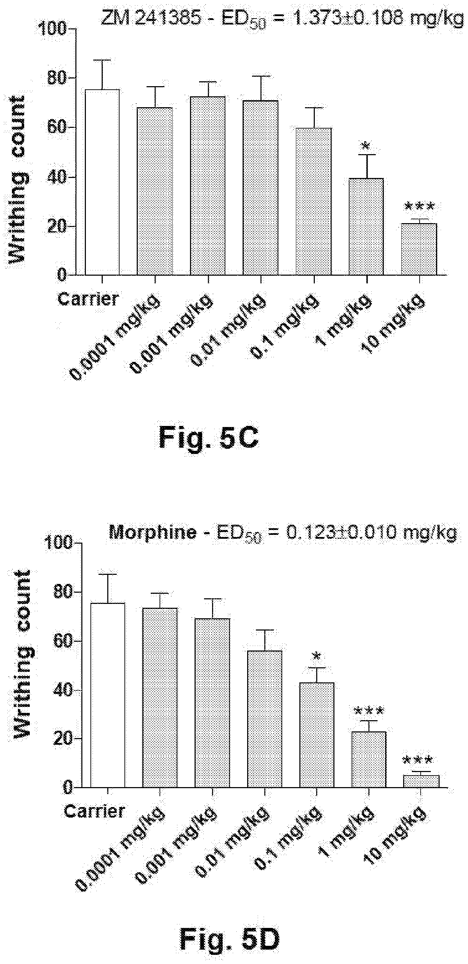

- Figures 5A, 5B, 5C, 5D show, in the form of histograms, the number of abdominal contractions induced by intraperitoneal administration of acetic acid in mice following treatment with carrier and with increasing doses of compound 1 (Fig. 5A) and of compound 3 (Fig. 5B) of the invention, and of the reference compounds ZM 241385 (Fig. 5C) and of morphine (Fig. 5D);

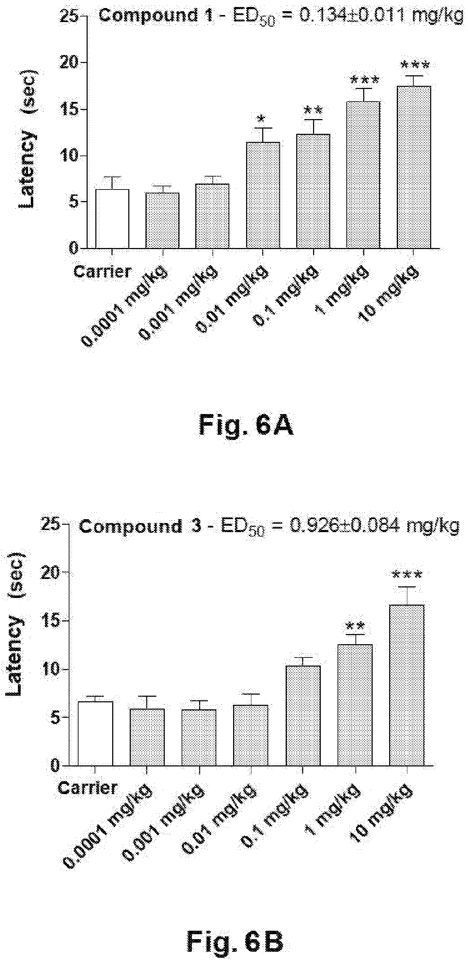

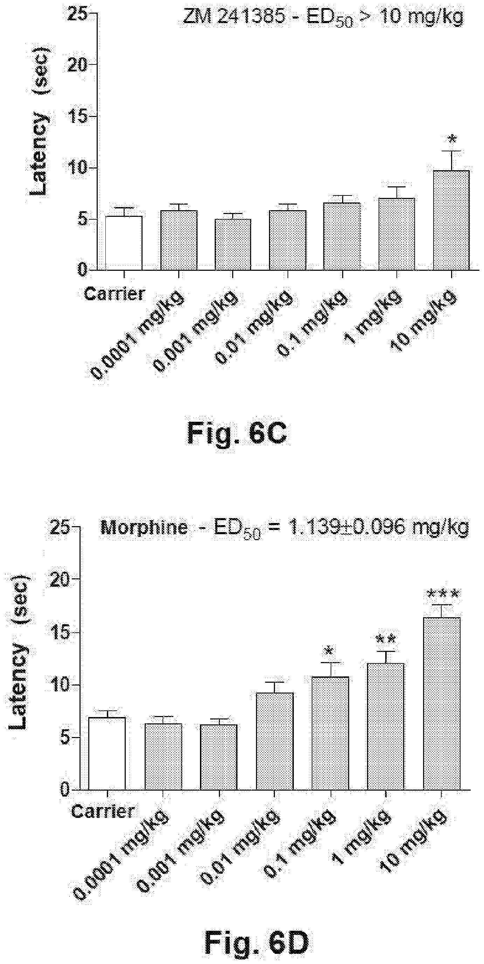

- Figures 6A, 6B, 6C and 6D show, in the form of histograms, the latency time of the tail withdrawal after immersion in hot water measured on mice following administration of carrier and increasing doses of compound 1 (Fig. 6A) and of the compound 3 (Fig. 6B) of the invention, and of the reference compounds ZM 241385 (Fig. 6C) and of morphine (Fig. 6D).

### Detailed description of the invention

In the present invention by the term "aryl" a monovalent aromatic hydrocarbon group is meant preferably having a single ring (for instance phenyl). Unless defined otherwise, these aryl groups typically contain from 6 to 10 carbon atoms in the ring. Preferred aryl groups comprise phenyl.

As used herein, the term "heteroaryl" refers to heteroaromatic groups, formed by a minimum of 5 to a maximum of 10 terms and containing from 1 to 3 heteroatoms, selected for instance from the group consisting of N, O, S, and oxidised derivatives; preferred heteroaryl groups comprise thienyl, furyl, and pyridyl.

As used herein, the term "alkyl" refers to a monovalent saturated hydrocarbon, which may be linear or branched. Representative alkyl groups include, as a non-limitative example, methyl, ethyl, n-propyl, isopropyl, n-butyl, sec-butyl, isobutyl, n-pentyl, n-hexyl, n-eptyl, n-octyl, n-nonyl, n-decyl, n-dodecyl, n-hexadecyl, and *n-*octadecyl. Methyl is a preferred alkyl group according to the invention.

By "optionally substituted" group a non-substituted group is meant or a group substituted with one or more substituents including nitro, cyano, halogen, amino, amido, oxo, carboxy, hydroxy, alkoxy, sulphoxy, or aliphatic chains, for example alkyl, alkenyl or alkynyl.

As used herein, the term "alkoxy" represents a monovalent group of formula (alkyl)-O-, wherein the term "alkyl" is defined as above and typically is methoxy, whereas the terms "alkenyl" and "alkynyl" refer to unsaturated hydrocarbon radicals, linear or branched, respectively with double or triple bonds.

As used herein, by the term "halogen" is meant fluoro, chloro, bromo or iodio.

Preferred compounds according to the present invention are the compounds of general formula (I) wherein R₃ is (CH₂)ₙaryl or (CH₂)ₙheteroaryl, optionally substituted, wherein n = 1 or 2.

The compounds of formula (I) as defined above can be prepared according to the synthetic scheme illustrated in the Scheme 1 illustrated below, and exemplified in the preparations of the compounds 1-17 of the following Examples 1-5: 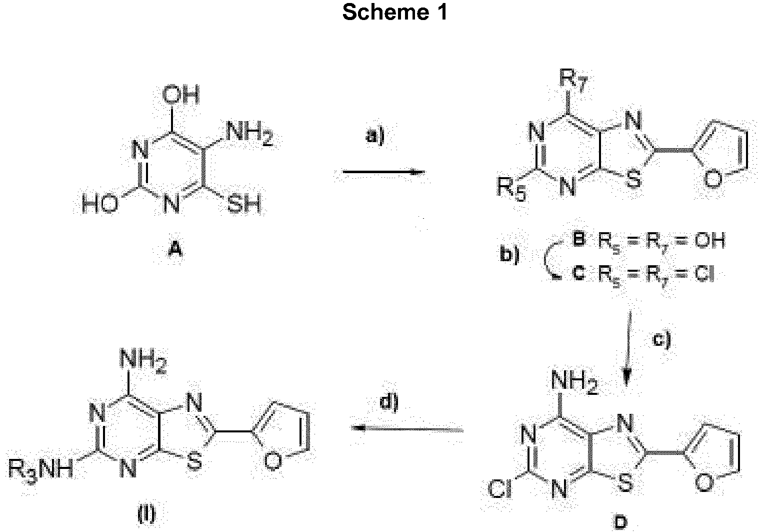 wherein R₃ is defined as above.

The preparation process of the Scheme 1 comprises therefore the following steps:
a) reaction of the starting compound A, 5-ammino-6-sulphanylpyrimidin-2,4-diol, with 2-furoylchloride to yield a compound B that is the 2-(furan-2-yl)-thiazolo[5,4-d]pyrimidin-5,7-diol;
b) chlorination of the compound B obtained in step a) with substitution of the two hydroxyl groups and formation of a 5,7-dichloro derivatives C;
c) substitution of chlorine at position 7 in the compound C obtained in step b) by its reaction with an aqueous solution of ammonia, to yield a compound D 7-ammino-5-chloro substituted; and
d) substitution of chlorine at position 5 in the compound D obtained in step c) with an ammine R₃NH₂ appropriately selected to obtain the compounds of formula (I) with the desired group R₃.

According to a preferred embodiment of the present invention, the process of Scheme 1 may be so accomplished:

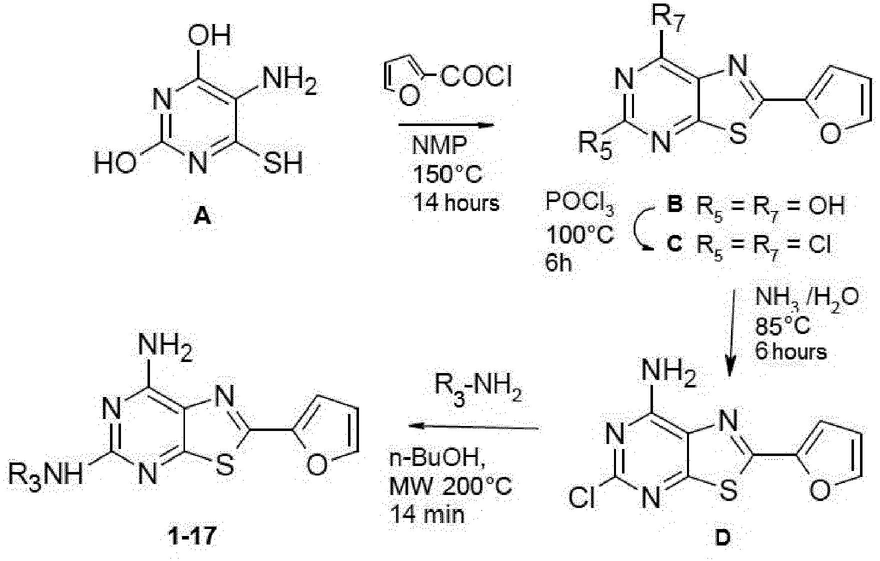

The process can therefore include heating of the starting compound A (G.P. Hager et al. J. Am. Pharm. As. 1955, 44, 193-196) easily synthesisable starting from commercial products, with 2-furoylchloride in N-methyl-2-pyrrolidone (NMP), so as to obtain with high yields the desired compound B, i.e. the 2-(furan-2-yl)-thiazolo[5,4-d]pyrimidin-5,7-diol. The so obtained compound B is treated with phosphoryl chloride to yield the 5,7-dichloro derivative C, which is then made to react with an aqueous solution of ammonia to yield the compound 7-ammino-5-chloro-substituted (compound D). The reaction of this latter, carried out by microwaves irradiation with a (hetero)arylalkyl or alkylamine, appropriately selected depending on the group R₃ to be introduced. The so obtained compounds bearing methoxy groups can then be transformed in the corresponding phenols by means of procedures known to any person skilled in the art, for instance by treatment with BBr₃ in CH₂Cl₂. By this process, as described in the Examples 1-5 reported below, are obtained the compounds 1-17 wherein R₃ is defined as follows:
1 R₃ = CH₂(2-OCH₃-C₆H₄)
2 R₃ = CH₂(4-OCH₃-C₆H₄) 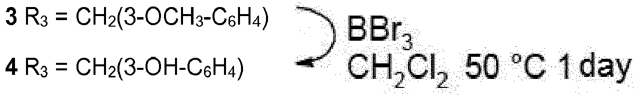
5 R₃ = CH₂(furan-2-yl)
6 R₃ = CH₂(thiophen-2-yl)
7 R₃ = CH₂(pyridin-3-yl)
8 R₃ = CH₂(pyridin-2-yl)
9 R₃ = CH₂(3-F-C₆H₄)
10 R₃ = CH₂CH₂(thiophen-2-yl)
11 R₃ = CH₂CH₂CH₃
12 R₃ = CH₂CH₂CH₂CH₃
13 R₃ = CH₂(thiophen-3-yl)
14 R₃ = CH₂(pyridin-4-yl)
15 R₃ = CH₂(pyrazin-2-yl)
16 R₃ = CH₂CH₂(furan-2-yl)
17 R₃ = CH₂CH₂(pyridin-3-yl)

If in the compounds of general formula (I) one or more asymmetric carbon atoms are present, the present invention comprises not only the respective pure enantiomeric forms, but also their scalemic or racemic mixtures. Moreover, if the compounds of general formula (I) exist in tautomeric forms, the present invention comprises any possible tautomeric forms.

In the present invention the term "pharmaceutically acceptable salt" refers to derivatives of the compound of formula (I) wherein the compound was appropriately modified by conversion of any acidic or basic group, if present, into the corresponding addition salt with any base or acid conventionally considered as acceptable for pharmaceutical use.

Suitable examples of these salts may include addition salts with organic or mineral acids of basic residues such as amine groups, or addition salts of acidic residues, such as carboxylic acids with bases such as those containing alkaline and alkaline earth metals (sodium, potassium, magnesium and calcium) or appropriate organic ammines. Possibly, the compounds of general formula (I) described in the present invention can form salts with aminoacids too.

The compounds of general formula (I) defined above according to the invention are useful in the treatment of diseases or disorders that are responsive to the blockade of adenosine A_{2A} receptors, and they can be used, alone or in combinations of two or more compounds, in pharmaceutical compositions with pharmaceutically acceptable carriers, excipients and/or diluents, and with possible further active principles. The present compounds can be present in the compositions as such or in the form of pharmaceutically acceptable salts.

The present pharmaceutical compositions can be formulated in several pharmaceutical forms, for different administration routes, for example as oral compositions or injectable solutions. They can moreover find application in the treatment of diseases or disorders associated to the activity of adenosine A_{2A} receptors, which can be treated therefore therapeutically thanks to the blockade of activity of the adenosine A_{2A} receptors. In other words, in the present invention, by "diseases or disorders associated or related to an activity of the adenosine A_{2A} receptor" are meant diseases or disorders that are responsive to the inhibition of an activity of the adenosine A_{2A} receptors, such as in particular neurologic diseases, pain, dermal fibrosis and scarring, and cancer.

### Experimental part

Chemistry. All reagents and solvents available on the market were purchased from Sigma Aldrich (Italy), and have been used without further purification. The microwave assisted synthesis were performed using an initiator EXP Microwave Biotage equipment (irradiation frequency: 2,45 GHz). Analytical silica gel plates (0.20 mm, F254, Merck, Germany), preparative silica gel plates (2 mm, F254, Merck, Germany) and silica gel 60 (70-230 mesh, Merck, Germany) were used for analytical and preparative TLC, and for column chromatography, respectively. Melting points were determined in glass capillary tubes on a Gallenkamp melting point apparatus. Compounds were named according to the IUPAC rules as applied by ACD/ChemSketch. Elemental analyses were performed with an elemental analyser for C, H and N Flash E1112 Thermofinnigan. IR spectra were recorded with a PerkinElmer Spectrum RX I spectrometer in Nujol dispersions and the data were expressed in cm⁻¹. Nuclear Magnetic Resonance experiments (NMR) were conducted on a Bruker Avance 400 (400 MHz for ¹H and 100 MHz for ¹³C NMR). The spectra were recorded at 300 K using DMSO-d6 as a solvent. Spectrum chemical shifts for ¹H and ¹³C were recorded in parts per million using residual non-deuterated solvent as internal standard. The following abbreviations are used: s = singlet, d = doublet, t = triplet, m= multiplet, br = broad, ar = aromatic protons, exch = exchangeable proton.

### EXAMPLE 1

### Preparation of 2-(furan-2-yl)[1,3]thiazolo[5,4-d]pyrimidin-5,7-diol (Compound B of the Scheme 1)

To a suspension of 5-ammino-6-sulphanylpyrimidin-2,4-diol (Compound A) (10 mmol) in anhydrous NMP, furan-2-carbonyl chloride (10 mmol) was slowly added. The resulting mixture was heated to 150°C under N₂ atmosphere for 14 hours. The reaction mixture was then cooled down to room temperature and diluted with cold water (100 ml) obtaining a precipitate that was then collected by filtration. Yield 82%. Pf: >300°C (DMSO). ¹H NMR: δ 6.73-6.74 (m, 1H, ar), 7.18-7.19 (m, 1H, ar), 7.93 (s, 1H, ar), 11.39 (br s, 1H, exch), 12.07 (br s, 1H, exch). ¹³C NMR: δ 110.75, 113.37, 130.71, 147.37, 147.95, 149.49, 150.47, 157.85. IR: 1673, 1703. Anal. calc. for C₉H₅N₃O₃S.

### EXAMPLE 2

### Preparation of 5,7-dichloro-2-(furan-2-yl)[1,3]thiazolo[5,4-d]pyrimidine (Compound C of the Scheme 1)

To a suspension in POCl₃ (20 ml) of the 5,7-diol (5 mmol) prepared as described above in the Example 1, was added at room temperature N,N-dimethylaniline (1.15 mL, 10 mmol). The resulting mixture was heated at 100°C for 6 hours. The organic phase was concentrated under vacuum, then the raw material was re-dissolved twice with cyclohexane (20 ml) and the organic portions evaporated under vacuum. The obtained residue was added with a mixture of water and ice (100 g), yielding a precipitate that was collected by filtration and used in the subsequent step without further purification. Yield 73%. ¹H NMR: δ 6.88-6.89 (m, 1H, ar), 7.65-7.66 (m, 1H, ar), 8.17 (s, 1H, ar). ¹³C NMR: δ 114.38, 116.23, 142.58, 147.02, 152.80, 152.84, 158.78, 167.66.

### EXAMPLE 3

### Preparation of 5-chloro-2-(furan-2-yl)[1,3]thiazolo[5,4-d]pyrimidin-7-ammine (Compound D of the Scheme 1)

A suspension of the 5,7-dichloro derivative (4 mmol) prepared as described above in the Example 2, in a mixture of 33% ammonia aqueous solution (15 ml) and ethanol (10 ml) was heated at 85°C for 6 hours. The reaction mixture was then cooled down to room temperature, obtaining a solid product, which was collected by filtration. Yield 75%. Pf: 296-300°C dec. (2-metoxyethanol/H₂O). ¹H NMR: δ 6.80-6.81 (m, 1H, ar), 7.29-7.30 (m, 1H, ar), 8.03 (s, 1H, ar), 8.27 (br s, 2H, exch). ¹³C NMR: δ 112.73, 113.62, 130.33, 147.70, 152.92, 155.35, 158.12, 163.05. IR: 3136, 3298. Anal. calc. for C₉H₅ClN₄OS.

### EXAMPLE 4

### General procedure for the preparation of the compounds 1-3 and 5-17

A (hetero)arylalkylamine or an alkylamine (3 mmol), appropriately selectable by any person with ordinary skills in the art depending on the final product to be obtained, was added to a solution in n-BuOH (2 ml) of the 5-chloro-7-amino derivative (1 mmol) prepared as described above in the Example 3. The reaction mixture was then irradiated with microwaves at 200°C for 20 minutes, then cooled down to room temperature and rendered basic with an aqueous solution of KOH (50%, 2 ml). The addition of water (approximately 100 ml) yielded a solid that was collected by filtration and washed with diethyl ether. The raw material was purified by crystallisation with organic solvents or by chromatography. The following compounds were so prepared and characterised:

### Compound 1 2-(furan-2-yl)-N⁵-(2-metoxybenzyl)[1,3]thiazolo[5,4-d]pyrimidin-5,7-diamine

Yield 81%. Pf: 254-256°C (acetic acid). ¹H NMR: δ 3.81 (s, 3H, OCH₃), 4.47 (d, 2H,CH₂, J = 6.2 Hz), 6.71-6.72 (m, 1H, ar), 6.87 (t, 1H, J = 7.3), 6.97 (d, 1H, ar, J = 7.8 Hz), 7.04-7.05 (m, 1H, ar), 7.14-7.24 (m, 5H, 2 ar + 3 exch), 7.89 (s, 1H, ar). ¹³C NMR: δ 55.69, 110.04, 110.67, 113.14, 120.51, 127.46, 128.00, 128.43, 148.62, 157.05, 157.56, 160.61. Anal. calc. for C₁₇H₁₅N₅O₂S.

### Compound 2 2-(furan-2-yl)-N⁵-(4-metoxybenzyl)[1,3]thiazolo[5,4-d]pyrimidin-5,7-diamine

Yield 68%. Pf: 189-192°C (EtOAc). ¹H NMR: δ 3.72 (s, 3H, OCH₃), 4.42 (d, 2H, CH₂, J = 5.8 Hz), 7.02-7.03 (m, 1H, ar), 6.86 (d, 2H, ar, J = 7.3 Hz), 7.04-7.05 (m, 1H, ar), 7.17 (br s, 2H, exch), 7.24-7.31 (m, 3H, 2 ar + 1 exch), 7.89 (s, 1H, ar). ¹³C NMR: δ 44.08, 55.47, 110.04, 113.16, 114.01, 128.83, 133.03, 148.61, 157.49, 158.46, 160.39. Anal. calc. for C₁₇H₁₅N₅O₂S.

### Compound 3 2-(furan-2-yl)-N⁵-(3-metoxybenzyl)[1,3]thiazolo[5,4-d]pyrimidin-5,7-diamine

Yield 70%. Pf: 199-201°C (EtOAc). ¹H NMR: δ 3.72 (s, 3H, OCH3), 4.46 (d, 2H, CH₂, J = 6.2 Hz), 6.71-6.72 (m, 1H, ar), 6.76-6.78 (m, 1H, ar), 6.88-6.89 (m, 2H, ar), 7.04-7.05 (m, 1H, ar), 7.19-7.23 (m, 3H, 1 ar + 2 exch), 7.32-7.38 (m, 1H, exch), 7.90 (s, 1H, ar). ¹³C NMR: δ 44.59, 55.39, 110.06, 112.24, 113.17, 119.70, 129.64, 142.83, 148.60, 157.51, 159.69, 160.42. Anal. calc. for C₁₇H₁₅N₅O₂S.

### Compound 5 2-(furan-2-yl)-N⁵-(furan-2-ylmethyl)[1,3]thiazolo[5,4-d]pyrimidin-5,7-diamine

Yield 73% Pf 220-224°C (chromatographic column with cyclohexane/ethyl acetate 3/7 as eluent) ¹H NMR: δ 4.47 (d, 2H, CH₂, J = 6.0 Hz), 6.24-6.25 (m, 1H, ar), 6.35-6.37 (m, 1H, ar), 6.71-6.72 (m, 1H, ar), 7.05-7.06 (m, 1H, ar), 7.23-7.27 (m, 3H, exch), 7.55 (s, 1H, ar), 7.90 (s, 1H, ar). Anal. calc. for C₁₄H₁₁N₅O₂S.

### Compound 6 2-(furan-2-yl)-N⁵-(thiophen-2-ylmethyl)[1,3]thiazolo[5,4-d]pyrimidin-5,7-diamine

Yield 89% Pf 192-196°C (isopropanol) ¹H NMR: δ 4.64 (d, 2H, CH₂, J = 6.2 Hz), 6.71-6.73 (m, 1H, ar), 6.93-6.95 (m, 1H, ar), 6.99-7.00 (m, 1H, ar), 7.06-7.07 (m, 1H, ar), 7.23 (s, 2H, exch), 7.32-7.33 (m, 1H, ar), 7.40 (t, 1H, exch, J = 6.2 Hz), 7.90 (s, 1H, ar). Anal. calc. for C₁₄H₁₁N₅OS₂.

### Compound 7 2-(furan-2-yl)-N⁵-(pyridin-3-ylmethyl)[1,3]thiazolo[5,4-d]pyrimidin-5,7-diamine

Yield 91% Pf 212-215°C (EtOH) ¹H NMR: δ 4.60 (d, 2H, CH₂, J = 6.1 Hz), 6.71-6.72 (m, 1H, ar), 7.04-7.05 (m, 1H, ar), 7.22-7.25 (m, 3H, 1 ar + 2 exch), 7.31 (d, 1H, ar, J = 7.9 Hz), 7.37 (br s, 1H, exch), 7.73 (t, 1H, ar, J = 7.6 Hz), 7.89 (s, 1H, ar), 7.19-7.23 (m, 3H, ar), 8.49-8.50 (m, 1H, ar). Anal. calc. for C₁₅H₁₂N₆OS.

### Compound 8 2-(furan-2-yl)-N⁵-(pyridin-2-ylmethyl)[1,3]thiazolo[5,4-d]pyrimidin-5,7-diamine

Yield 94% Pf 213-217°C (EtOH) ¹H NMR: δ 4.62 (d, 2H, CH₂, J=5.9 Hz), 6.71-6.74 (m, 1H, ar), 7.05-7.09 (m, 1H, ar), 7.25-7.40 (m, 5H, 2ar + 3 exch), 7.77-7.81 (m, 1H, ar), 7.87-7.90 (m, 1H, ar), 8.50-8.52 (m, 1H, ar). Anal. calc. for C₁₅H₁₂N₆OS.

### Compound 9 N⁵-(3-fluorobenzyl)-2-(furan-2-yl)[1,3]thiazolo[5,4-d]pyrimidin-5,7-diamine

Yield 64% Pf 217-221°C (nitromethane) ¹H NMR: δ 4.51 (d, 2H, CH₂, J = 6.0 Hz), 6.71-6.72 (m, 1H, ar), 7.01-7.22 (m, 6H, 4ar + 2exch), 7.32-7.37 (m, 1H, ar), 7.40-7.42 (m, 1H, exch), 7.89-7.91(s, 1H, ar). Anal. calc. for C₁₆H₁₂FN₅OS.

### Compound 10 2-(furan-2-yl)-N⁵-(2-(thiophen-2-yl)ethyl)-[1,3]thiazolo[5,4-d]pyrimidin-5,7-diamine

Yield 78% Pf 216-218°C (nitromethane) ¹H NMR: δ 3.06 (t, 2H, CH₂, J = 7.2 Hz), 3.51(dd, 2H, CH₂, J =13.4, 7.0 Hz), 6.71-6.72 (m, 1H, ar), 6.92-6.96 (m ,3H, ar), 7.04-7.05 (m, 1H, ar), 7.18 (br s, 2H, exch), 7.32-7.34 (m, 1H, exch), 7.89-7.90 (m, 1H, ar). Anal. calc. for C₁₅H₁₃N₅OS₂.

### Compound 11 2-(furan-2-yl)-N⁵-propyl-[1,3]thiazolo[5,4-d]pyrimidin-5,7-diamine

Yield 65% Pf 204-207°C (EtOAc) ¹H NMR: δ 0.87-0.91 (m, 3H, CH₃), 1.50-1.55 (m, 2H, CH₂), 3.18-3.22 (m, 2H, CH₂), 6.71-6.72 (m, 1H, ar), 6.83 (br s, 1H, exch), 7.03-7.04 (m, 1H, ar), 7.10 (br s, 2H, exch), 7.89-7.90 (m 1H, ar). Anal. calc. for C₁₂H₁₃N₅OS.

### Compound 12 N⁵-butyl-2-(furan-2-yl)[1,3]thiazolo[5,4-d]pyrimidin-5,7-diamine

Yield 70% Pf 201-205°C (EtOAc) ¹H NMR: δ 0.85-0.90 (m, 3H, CH₃), 1.32-1.35 (m, 2H, CH₂) 1.48-1.52 (m, 2H, CH₂), 3.24-3.26 (m, 2H, CH₂), 6.71-6.72 (m, 1H, ar), 6.81 (br s, 1H, exch), 7.03-7.04 (m, 1H, ar), 7.10 (br s, 2H, exch), 7.88-7.89 (m, 1H, ar). Anal. calc. for C₁₃H₁₅N₅OS.

### Compound 13 2-(furan-2-yl)-N⁵-(thiophen-3-ylmethyl)[1,3]thiazolo[5,4-d]pyrimidin-5,7-diamine

Yield 74 % Pf: 209-212 °C (EtOAc). ¹H NMR: δ 4.47 (d, 2H, CH₂, J= 6.1 Hz), 6.72 (dd, 1H, ar, J= 3.3 Hz, 1.7 Hz), 7.05-7.06 (m, 1H, ar), 7.10 (broad s, 1H, exch), 7.22 (broad s, 2H, exch), 7.29-7.32 (m, 2H, ar), 7.45 (dd, 1H, ar, J= 4.8 Hz, 3.0 Hz), 7.89-7.90 (m, 1H, ar). Anal calc. for C₁₄H₁₁N₅OS₂

### Compound 14 2-(furan-2-yl)-N⁵-(pyridin-4-ylmethyl)[1,3]thiazolo[5,4-d]pyrimidin-5,7-diamine

Yield 72 % Pf: 223-225°C (preparative plate with ethyl acetate/methanol 9/2.5 as eluent). ¹H NMR: 4.51 (d, 2H, CH₂, J= 6 Hz) 6.71-6.72 (m, 1H, ar), 7.04-7.05 (m, 1H, ar), 7.23 (s, 2H, exch), 7.30-7.31 (m, 2H, ar), 7.47 (broad s, 1H, exch), 7.89-7.90 (m, 1H, ar), 8.47-8.48 (m, 2H, ar) δ Anal calc. for C₁₅H₁₂N₆OS.

### Compound 15 2-(furan-2-yl)-N⁵-(pyrazin-2-ylmethyl)[1,3]thiazolo[5,4-d]pyrimidin-5,7-diamine

Yield 68 % Pf: 236-238°C (acetic acid/EtOH). ¹H NMR: δ 4.63 (d, 2H, CH₂, J= 6.1 Hz), 6.72 (dd, 1H, ar, J= 3.4 Hz, 1.7 Hz), 7.05-7.06 (m, 1H, ar), 7.27 (s, 2H, exch), 7.48 (broad s, 1H, exch), 7.89-7.90 (m, 1H, ar), 8.50-8.51 (m, 1H, ar), 8.57-8.58 (m, 1H, ar), 8.62 (s, 1H, ar). Anal calc. for C₁₄H₁₁N₇OS

### Compound 16 2-(furan-2-yl)-N⁵-(2-(furan-2-yl)ethyl)[1,3]thiazolo[5,4-d]pyrimidin-5,7-diamine

Yield 86 % Pf: 198-200°C (chromatographic column with ethyl acetate/cyclohexane 1/1 as eluent). ¹H NMR: δ 2.87 (t, 2H, CH₂, J= 7.0 Hz), 3.51-3.53 (m, 2H, CH₂), 6.17-6.18 (m, 1H, ar), 6.35-6.36 (m, 1H, ar), 6.71-6.72 (m, 1H, ar), 6.90 (broad s, 1H, exch), 7.04-7.05 (m, 1H, ar), 7.17 (broad s, 2H, exch), 7.51-7.52 (m, 1H, ar), 7.88-7.89 (m, 1H, ar). Anal calc. for C₁₅H₁₃N₅O₂S

### Compound 17 2-(furan-2-yl)-N⁵-(2-(pyridin-3-yl)ethyl)[1,3]thiazolo[5,4-d]pyrimidin-5,7-diamine

Yield 59 % Pf: 176-178°C (preparative plate with ethyl acetate/cyclohexane/methanol 8/1/1 as eluent). ¹H NMR: δ 2.87 (t, 2H, CH₂, J= 7.0 Hz), 3.50 (dd, 2H, CH₂, J= 12.9 Hz, 6.7 Hz), 6.72-6.73 (m, 1H, ar), 6.95 (broad s, 1H, exch), 7.04-7.05 (m, 1H, ar), 7.18 (slargato s, 2H, exch), 7.30 (dd, 1H, ar, J= 7.6 Hz, 4.7 Hz), 7.68 (d, 1H, ar, J= 6.6 Hz), 7.89-7.90 (m, 1H, ar), 8.40 (d, 1H, ar, J= 4.7 Hz), 8.47 (s, 1H, ar). Anal calc. for C₁₆H₁₄N₆OS

### EXAMPLE 5

### Preparation of 3-(((7-amino-2-(furan-2-yl)[1,3]thiazolo[5,4-d]pyrimidin-5-yl)amino) methyl) phenol (Compound 4)

A solution of BBr₃ in CH₂Cl₂ (1 M, 1.5 ml) was added drop by drop to a suspension in anhydrous dichloromethane (40 ml) of the Compound 3 (0.5 mmol) prepared as described above in the Example 4. Upon completion of the addition, the suspension was maintained under stirring at 50°C for 1 day. The solution was diluted with water and ice (50 g) and maintained under stirring for 4 hours, then a saturated aqueous solution of NaHCO₃ (6 ml) was added. The resulting precipitate was collected by filtration and washed with water. Yield 80%. Pf: 207-209°C (EtOAc). ¹H NMR: δ 4.44 (d, 2H, CH₂, J = 5.3 Hz), 6.58 (d, 1H, ar, J = 8.9 Hz), 6.68-6.71 (m, 3H, ar), 7.04-7.09 (m, 2H, ar), 7.23 (br s, 2H, exch), 7.35 (br s, 1H, exch), 7.89 (s, 1H, ar), 9.25 (br s, 1H, exch). ¹³C NMR: δ 44.45, 110.06, 113.17, 113.81, 114.13, 117.97, 129.54, 142.65, 145.52, 148.59, 157.50, 157.76, 160.35, 164.86. Anal. calc. for C₁₆H₁₃N₅O₂S.

### EXAMPLE 6 - Pharmacologic Tests

### In vitro pharmacological tests

### Cell culture and membrane preparation

Chinese Hamster Ovary (CHO) cells transfected with human (h) adenosine receptors A₁, A_{2A}, A_{2B} and A₃ were cultured and maintained in Dulbecco's modified with a mixture of nutrients F12, containing the 10% of bovine foetal serum, penicillin (100 U/ml), streptomycin (100 µg/ml), I-glutamine (2 mM), geneticin (G418; 0.2 mg/ml) at 37°C in 5% CO₂ and 95% of air until use in cAMP assays. For the membranes preparation the culture medium was removed, and the cells were washed with a saline phosphate-buffered solution collected with hypotonic buffer (5 mM Tris HCl, 1 mM EDTA, pH 7.4). The cell suspension was homogenised with a Polytron, centrifuged for 30 minutes at 40000 g at 4°C and the resulting membrane pellet was used in competition binding experiments (Varani K, et al., Mol. Pharmacol. 2000; 57:968-975).

### Competition binding experiments

The compounds of the present invention and the known compound ZM 241385 as reference were tested for their affinity to the following human receptors of adenosine: hA₁, hA_{2A} and hA₃. Competition binding experiments for the receptor A₁ were carried out incubating 1 nM [³H]-DPCPX with the membrane suspension (50 µg of protein/100 µl) and different concentrations of the compounds evaluated at 25°C for 90 minutes in 50 mM Tris HCl, pH 7.4. Non-specific binding was defined as binding in the presence of 1 µM DPCPX and was always <10% of the total binding. Inhibition experiments to A_{2A} receptors were carried out by incubating the radioligand [³H]-ZM 241385 (1 nM) with the membrane suspension (50 µg of protein/100 µl) and at least 12 different concentrations of the tested compounds for 60 minutes at 4°C in 50 mM Tris HCI (pH 7.4), 10 mM MgCl₂. Non-specific binding was determined in the presence of ZM 241385 (1 µM) and was approximately the 20% of the total binding.

Competition binding experiments for the binding to the A₃ receptor were carried out by incubating the membrane suspension (50 µg of protein/100 µl) with 0.5 nM [¹²⁵I]-ABMECA in the presence of the compounds under evaluation at various concentrations for an incubation time of 120 minutes at 4°C in 50 mM Tris HCI (pH 7.4), 10 mM MgCl₂, 1 mM EDTA. The non-specific binding was defined as binding in the presence of 1 µM ABMECA and was always <10% of the total binding.

Bound radioactivity and free radioactivity were separated by filtering the solution through Whatman GF/B glass fibre filters using a Brandel cell harvester (Brandel Instruments, Unterföhring, Germany). The filter-bound radioactivity was counted by a scintillation counter Packard Tri Carb 2810 TR (Perkin Elmer) (Varani K. et al., Mol. Pharmacol. 2000;57:968-975).

### Cyclic AMP assays

CHO cells transfected with the adenosine receptors were washed with a phosphate-buffered saline solution, detached with trypsin and centrifuged for 10 minutes at 200 g. The pellet containing CHO cells (1x10⁶ cells/sample) was suspended in 0.5 ml of the incubation mixture (mM): NaCl 15, KCI 0.27, NaH₂PO₄ 0.037, MgSO₄ 0.1, CaCl₂ 0.1, Hepes 0.01, MgCl₂ 1, glucose 0.5, pH 7.4 at 37°C, 2 IU/ml adenosine deaminase and 4-(3-butoxy-4-metoxybenzyl)-2-imidazolidinone (Ro 20-1724) as phosphodiesterase inhibitor and pre-incubated for 10 minutes in a thermostated bath under stirring at 37°C. The potencies of the evaluated compounds for the hA_{2B} receptors have been determined by evaluating their ability to inhibit the cAMP levels stimulated by NECA (100 nM). In order to better investigate the behaviour of inverse antagonism/agonism, the potency towards the hA_{2A} receptors of the compounds tested at 12 different concentrations was determined by studying their ability to inhibit the production of cAMP both under basal conditions and in the presence of the known agonist CGS 21680 (10 nM). Additional experiments have been carried out by evaluating the studied compounds at the concentration of 10 µM in hA₁, hA_{2B} or hA₃CHO cells in order to verify their effect on the cAMP production under basal conditions. The reaction was terminated by addition of cold 6% trichloroacetic acid (TCA). The TCA suspension was centrifuged at 2000 g for 10 minutes at 4°C and the supernatant was extracted four times with diethyl ether with water. The final aqueous solution was tested for the cAMP levels by a competition protein binding assay with a cAMP-binding protein. The standard samples of cAMP (0-10 pmoli) were added to each test tube containing the incubation buffer (trizma base 0.1 M, aminophylline 8.0 mM, 2 mercaptoethanol 6.0 mM, pH 7.4) and [³H]-cAMP. The binding protein previously prepared from beef adrenals, was added to the samples previously incubated at 4°C for 150 minutes, and after the addition of charcoal were centrifuged at 2000 g for 10 min. The transparent surnatant was counted in a 2810-TR Packard scintillation counter (Varani K et al., Biochem Pharmacol 2005;70:1601-1612).

### Cell proliferation assay

For the experiments of cellular proliferation, the DELFIA® kit was used and a multimode plate reader Ensight® from PerkinElmer. The DELFIA® (*dissociation-enhanced lanthanide fluorescence immunoassay*) assay is based on Time-Resolved Fluorescence (TRF) and on the incorporation of the 5-bromo-2-deoxyuridine (BrdU) in the DNA filaments recently synthesised by the proliferating cells seeded on microplates. The incorporated BrdU is detected by using a monoclonal antibody conjugated with europium, a long-lived chelated lanthanide, and the fluorescence measured is proportional to the synthesis of DNA in the cells population present in each well. The MRMT-1 cells, breast cancer cells, were pre-treated with some of the antagonists under evaluation at the concentration of 100 nM (compounds 1, 5, 6, 7, 10 of the present invention) for 30 minutes, then stimulated with CGS 21680 100 nM and after 30 minutes the solution of BrdU (10 µl/well) was added. At the end of the incubation period of 48 hours 100 µl/well of Anti-BrdU-Eu (0.5 µg/ml) were added and the cells were incubated for 120 minutes at room temperature. After 4 washings, 200 µl of DELFIA® stimulator were added at room temperature for 15 minutes and the Eu-fluorescence was detected by means of the Ensight® reader from Perkin Elmer (Perkin Elmer, Milan, Italy).

### In vivo pharmacological tests

### Animals

Female CD1 mice (22-24 g) were obtained from Charles River (Milano, Italia). The animals were kept under standard environmental temperature conditions (22±2°C) and under moisture-controlled conditions with 12 hours light/dark cycle and food and water *ad libitum.* The animals were acclimated to the laboratory settings for at least 1 hour before testing and were used only once throughout the experiments. All the procedures used in the present study were carried out in accordance with the European Communities Council Directives (86/609/EEC) and the National Laws and Policies (D.L.116/92) after authorization from the Italian Ministry for Health (4/2014-B). In addition, the experimental procedures were in agreement with the current guidelines for the care of laboratory animals and the ethical guidelines for investigations of experimental pain in conscious animals (Couto M. Methods Mol Biol 2011;770:579-599).

### Writhing Test

The acetic acid-induced writhing response was performed after intraperitoneal injection of 10 ml/Kg of 0.6% acetic acid solution. The response to the abdominal constrictions induced by acetic acid was evaluated after the intraperitoneal injection of 10 ml/kg of a 0.6% solution of acetic acid. The compounds under evaluation were dissolved in DMSO and then diluted in a saline solution. The carrier consists of saline solution and 5% of DMSO. A writhe is indicated by stretching of the abdomen followed by the extension of the hind limbs. The animals (8 mice per group) were placed singly in a glass cylinder and the number of writhing episodes of abdominal contractions was counted in a 30 minutes period. The compounds were administered intraperitoneally 15 minutes before injection of acetic acid solution. As expected, no abdominal constrictions were observed in mice treated with saline solution instead of with acetic acid solution (Vincenzi F. et al., Pain 2013;154:864-873). The values of ED₅₀ were calculated by a linear regression analysis converting the data to percentage of maximum possible effect (MPE) using the following equation: 100 X (post drug response - response to carrier)/(response to carrier).

### Tail immersion test

The warm-water tail immersion assay was performed using a water thermostated bath at a temperature maintained at 52°C. The compounds under evaluation were dissolved in DMSO and then diluted in saline solution. The carrier consists of saline solution and 5% of DMSO. Before intraperitoneally injecting the compound, the natural time of response of the mice was determined and the distal part of the tail was then immersed in the thermostated bath. The latency in responding to the heat stimulus with a vigorous flexion of the tail was measured by means of a manual stopwatch. A 20 seconds maximum cut-off time was imposed to prevent tissue damage. The latency of the tail withdrawal was then tested 15 minutes after injection of the compound. The values of ED₅₀ were calculated by a linear regression analysis converting the data to %MPE using the following equation: 100 X (post-drug latency - basal latency) / (cut-off latency - basal latency).

### Statistical analysis of the data

The statistical analysis of the data was performed using ANOVA followed by Dunnett's test. The inhibitory binding constants, Ki, will be calculated from the IC50 values according to the Cheng e Prusoff equation: Ki = IC₅₀/(1 + [C*]/KD*), wherein [C*] is the radioligand concentration and KD* its dissociation constant. KH and KL were obtained by using a two sites binding model and Graph PAD Prism (San Diego, CA, USA). The values of IC₅₀ obtained in the cAMP assays were calculated by non-linear regression analysis using the equation for a sigmoid concentration-response curve. All data are expressed as the mean ± SEM of four independent experiments each performed in duplicate for *in vitro* assays and n = 8-10 mice/group for *in vivo* assays.

### Results of the in vitro tests

### Evaluation of affinity at human adenosine A₁, A_{2A} and A₃ receptors

Affinity at human adenosine A₁, A_{2A} and A₃ receptors of the tested compounds of the present invention expressed as Ki values are listed in the Table 1 below, together with those of ZM 241385 as the reference compound.

**Table 1**

| **Compound** | **Receptor hA₁^{[a]}** | **Receptor hA_{2A}^{[b]}** | **Receptor hA₃^{[c]}** | **Receptor hA_{2B}^{[d]}** |
|---|---|---|---|---|
| | **Ki (nM)** | **Ki (nM) or KH* (fM) and KL** (nM)** | **Ki (nM)** | **IC₅₀ (nM)** |
| 1 | 3.54±0.32 | 3.55±0.42* 6.45±0.57** | 36±3 | 313±29 |
| 2 | 163±12 | 171±16 | 381±37 | 283±27 |
| 3 | 8.16±0.72 | 5.31±0.52* 26±2** | 92±8 | 452±42 |
| 4 | 27±3 | 20±2 | 55±4 | 24±3 |
| 5 | 38±4 | 39±4* 1.73±0.15** | 4.72±0.38 | 82±9 |
| 6 | 12.5±1.1 | 10.7±1.0* 3.82±0.31** | 6.43±8 | 75±8 |
| 7 | 7.12±0.65 | 217±19* 0.68±0.05** | 18.2±1.7 | 109±11 |
| 8 | 28±3 | 0.42±0.04 | 59±5 | 95±9 |
| 9 | 8.51±0.76 | 0.82±0.07 | 35±4 | 103±10 |
| 10 | 4.92±0.37 | 10.6±0.9* 18±2** | 65±6 | 112±11 |
| 11 | 64±10 | 17±2 | 35±4 | 323±28 |
| 12 | 41±5 | 8.21±0.78 | 23±3 | 185±17 |
| 13 | 8.12±0.71 | 0.25±0.02 | 3.14±0.29 | 8.96±0.82 |
| 14 | 47±4 | 12±1 | 827±48 | 33±2 |
| 15 | 25±4 | 5.14±0.48 | 157±14 | 13.2±1.2 |
| 16 | 5.24±0.46 | 2.15±0.19 | 23±2 | 14±1 |
| 17 | 2.61±0.22 | 0.24±0.01 | 174±11 | 4.21±0.32 |
| ZM 241385 | 185±14 | 0.91±0.08 | 683±64 | 48±5 |

| | | | | |
|---|---|---|---|---|
| Affinity values obtained from competition binding experiments using [³H]-DPCPX [a], [³H]-ZM 241385 [b] or [¹²⁵I]-ABMECA [c] binding to human adenosine A₁, A_{2A}, A₃ receptors respectively (n=3-6). [d] Potency (IC₅₀) in cAMP assays to human adenosine A_{2B} receptor. Data are expressed as mean ± SEM. | | | | |

It is worth noting that in the competition binding experiments for the binding with [³H]-ZM 241385, the compounds 1, 3, 5, 6, 7 and 10 of the invention showed two affinity values for the human adenosine A_{2A} receptor, the first one having a high value of the affinity Ki (KH) of the femtomolar order and the second one having a nanomolar affinity value Ki (KL) (Table 1). It is also worth noting that the KH values of these compounds are approximately 10⁶ times lower than their corresponding KL values. On the contrary, in competition binding experiments the compounds 2, 4, 8, 9, 11-17 and ZM 241385, showed only an affinity value Ki in the nanomolar order. The competition binding curves of the compounds 1, 3, 5, 6, 7 and 10 showed a biphasic form that better match with a two sites binding model and can be interpreted as the interaction with two apparent binding sites whilst the competition binding curves of the reference compound ZM 241385 indicated the presence of a binding site recognition(Fig. 1).

### Potency values at the human adenosine receptors

Also studied was the *in vitro* activity of the compounds according to the present invention by evaluating their antagonist/inverse agonist potencies. In particular, the ability was tested for the compounds 1-8, 10, 13-17 and ZM 241385 to modulate the cAMP production in hA_{2A} CHO cells in the absence or in presence of CGS 21680. The potency values and the efficacy values of the tested compounds in comparison with ZM 241385 are listed in the Table 2 below. According to their extremely high affinity for the human adenosine A_{2A} receptor, the compounds 1, 3, 5, 6, 7 and 10 behaved as very potent inverse agonists, being able to inhibit the basal accumulation of cAMP at picomolar concentrations (IC₅₀=1.9, 8.3, 1.6, 1.7, 11 and 6.4 pM, respectively) (Table 2), and showing efficacy values of 63%, 41%, 64%, 61%, 61% and 62% respectively (Fig. 2A, 2B, 2C, 2D, 2E, 2F).

It is worth noting that the compounds 1, 3, 5, 6 , 7 and 10 behaved as inverse agonists having higher potencies with respect to ZM 241385 (IC₅₀=1.45 nM) (Fig. 2G). The compounds 2, 4, 8 and 13-17 significantly reduced the cAMP production in basal conditions with IC₅₀ values comprised between 187 and 0.29 nM (Table 2). Moreover, the compounds 1, 3, 5, 6, 7 and 10 were also able to inhibit the cAMP production stimulated by CGS 21680 (10 nM) with high potency (IC₅₀=51, 95, 40, 36, 59, 45 pM, respectively) (Fig. 3A, 3B, 3C, 3D, 3E, 3F; Table 2). The reference compound ZM 241385 blocked the effect of the agonist with a lower potency (IC₅₀= 678 pM, Fig. 3G) than those of the compounds 1, 3, 5, 6, 7 and 10.

As expected, all the tested compounds in the presence of an agonist showed an antagonist/inverse agonist profile. In particular, they reduced the cAMP accumulation, reaching lower values than those of the basal production as indicated in the Emax data (Fig. 3; Table 2). In functional assays performed in CHO cells transfected with human adenosine A₁, A_{2B} and A₃ receptors, none of the compounds under evaluation were able to modulate the cAMP production in the absence of an agonist, suggesting that at these receptor subtypes the tested compounds do not behave as inverse agonists.

**Table 2**

| **Compounds** | **IC₅₀ (nM)^{[a]}** | **Emax (%)^{[b]}** | **IC₅₀ (nM)^{[c]}** | **Emax (%)^{[d]}** |
|---|---|---|---|---|
| 1 | 0.0019±0.0002 | 63±5 | 0.051±0.004 | 138±12 |
| 2 | 187±16 | 38±4 | 123±11 | 116±11 |
| 3 | 0.0083±0.0007 | 41±3 | 0.095±0.008 | 136±11 |
| 4 | 27±2 | 68±7 | 22±2 | 139±13 |
| 5 | 0.0016±0.0002 | 64±6 | 0.040±0.004 | 133±13 |
| 6 | 0.0017±0.0002 | 61±6 | 0.036±0.003 | 132±12 |
| 7 | 0.011±0.001 | 61±5 | 0.059±0.006 | 126±12 |
| 8 | 0.68±0.06 | 54±5 | 1.3±0.1 | 128±11 |
| 10 | 0.0064±0.0005 | 62±6 | 0.045±0.004 | 138±13 |
| 13 | 0.36±0.04 | 67±6 | 0.41±0.03 | 137±13 |
| 14 | 15.3±1.2 | 43±4 | 18.7±1.6 | 122±10 |
| 15 | 8.27±0.72 | 48±4 | 11.3±0.96 | 117±10 |
| 16 | 2.93±0.22 | 56±6 | 4.26±0.37 | 121±11 |
| 17 | 0.29±0.03 | 71±7 | 0.35±0.04 | 141±14 |
| ZM 241385 | 1.45±0.42 | 46±2 | 0.678±0.061 | 123±10 |

| | | | | |
|---|---|---|---|---|
| Potency (IC₅₀) [a,c] and efficacy (Emax) [b,d] of the tested compounds in cAMP assays in hA_{2A} CHO cells in absence [a,b] or in presence [c,d] of CGS 21680 (10 nM), respectively. The data are expressed as mean ± SEM. | | | | |

### Results of cellular proliferation

The cellular proliferation assays performed on the breast cancer cells MRMT-1 expressing the adenosine receptors have showed that CGS 21680 is able to increase the proliferation of the cancer cells. The effect of the agonist CGS 21680 is blocked by the use of the compounds 1, 5, 6, 7 and 10 of the present invention that are able to reduce the proliferation of cancer cells induced by the activation of the adenosine A_{2A} receptor (Fig. 4)

### In vivo results

### Analgesic effects of the novel compounds of the invention

To explore the anti-nociceptive activity of the novel inverse agonists of the human adenosine A_{2A} receptor, the compounds 1 and 3 have been tested in comparison with ZM 241385 and with morphine in mice, in an evaluation test of the abdominal constrictions (Writhing Test) and in a tail immersion test. In the Writhing Test, the intraperitoneal administration of acetic acid induced 75±12 abdominal constrictions in mice treated with a carrier. The dose-response curve of compounds 1, 3, ZM 241385 and morphine revealed a dose-dependent effect (P<0.001, one-way ANOVA). In particular, the compound 1 of the present invention proved to be more potent than the reference compounds. In fact, it shows an ED₅₀ value of 0.0328±0.0021 mg/kg, which is 3.75 times lower than that obtained with morphine (0.123±0.010 mg/kg) and approximately 42 times lower than that of ZM 241385 (1.373±0.108 mg/kg) (Fig. 5A-D). The compound 3 of the present invention had an anti-nociceptive activity with potency similar to morphine. In the warm water tail immersion test, the compound 1 of the present invention shoed the highest analgesic activity with an ED₅₀ value of 0.134±0.011 mg/kg and a minimum effective dose of 0.01 mg/kg. The compound 3 of the present invention and morphine showed similar anti-nociceptive activity whereas ZM 241385 had no effect up to 10 mg/kg (Fig. 6A-D).

## Claims

1. Compounds of general formula (I) 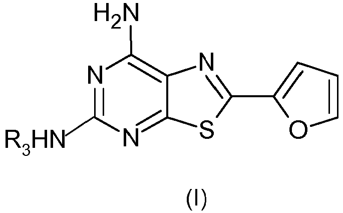 wherein R₃ is selected from the group consisting of hydrogen, alkyl optionally substituted, (CH₂)ₙaryl optionally substituted and (CH₂)ₙheteroaryl optionally substituted, wherein n is an integer ranging from 0 to 4,
and pharmaceutically acceptable salts, tautomers and enantiomers thereof.

2. The compounds according to the claim 1, wherein R₃ is (CH₂)ₙaryl or (CH₂)ₙheteroaryl, optionally substituted, wherein n = 1 o 2.

3. The compounds according to any one of the preceding claims, selected from the group consisting of:
2-(furan-2-yl)-N⁵-(2-metoxybenzyl)[1,3]thiazolo[5,4-d]pyrimidin-5,7-diamine
2-(furan-2-yl)-N⁵-(4-metoxybenzyl)[1,3]thiazolo[5,4-d]pyrimidin-5,7-diamine
2-(furan-2-yl)-N⁵-(3-metoxybenzyl)[1,3]thiazolo[5,4-d]pyrimidin-5,7-diamine
3-(((7-amino-2-(furan-2-yl)[1,3]thiazolo[5,4-d]pyrimidin-5-yl)amino)methyl) phenol
2-(furan-2-yl)-N⁵-(furan-2-ylmethyl)[1,3]thiazolo[5,4-d]pyrimidin-5,7-diamine
2-(furan-2-yl)-N⁵-(thiophen-2-ylmethyl)[1,3]thiazolo[5,4-d]pyrimidin-5,7-diamine
2-(furan-2-yl)-N⁵-(pyridin-3-ylmethyl)[1,3]thiazolo[5,4-d]pyrimidin-5,7-diamine
2-(furan-2-yl)-N⁵-(pyridin-2-ylmethyl)[1,3]thiazolo[5,4-d]pyrimidin-5,7-diamine
N⁵-(3-fluorobenzyl)-2-(furan-2-yl)[1,3]thiazolo[5,4-d]pyrimidin-5,7-diamine
2-(furan-2-yl)-N⁵-(2-(thiophen-2-yl)ethyl)-[1,3]thiazolo[5,4-d]pyrimidin-5,7-diamine
2-(furan-2-yl)-N⁵-propyl-[1,3]thiazolo[5,4-d]pyrimidin-5,7-diamine, and
N⁵-butyl-2-(furan-2-yl)[1,3]thiazolo[5,4-d]pyrimidin-5,7-diamine.

4. A pharmaceutical composition comprising as the active principle at least a compound of general formula (I) as defined in the claims from 1 to 3, in admixture with one or more excipients and/or diluents and/or pharmaceutically acceptable carriers.

5. The pharmaceutical composition according to claim 4, further comprising one or more further active principles.

6. Compounds according to any one of the claims from 1 to 3, for use as medicaments.

7. Compounds according to any one of the claims from 1 to 3, for use in the therapeutic treatment of diseases or disorders responsive to the blockade of the adenosine A_{2A} receptor.

8. Compounds for use according to claim 7, wherein said diseases or disorders are selected from the group consisting of neurological pathologies, pain, cancer, dermal fibrosis and scarring.

9. A process for the preparation of the compounds of general formula (I) as defined in the claims from 1 to 3, comprising the following steps according to the scheme illustrated below: 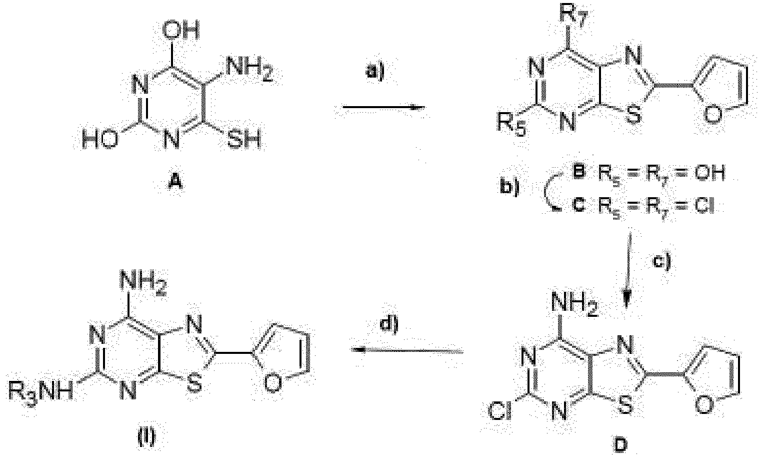 wherein R₃ is defined as in the claims 1-3:
a) reaction of the starting compound A, 5-amino-6-sulphanylpyrimidin-2,4-diol, with 2-furoylchloride to form a compound B, which is the 2-(furan-2-yl)-thiazolo[5,4-d]pyrimidin-5,7-diol;
b) chlorination of the compound B obtained in step a) with substitution of the two hydroxyl groups and formation of a 5,7-dichloro derivative C;
c) substitution of chloro at position 7 in the compound C obtained in step b) by reacting it with an aqueous solution of ammonia, to form a compound D 7-amino-5-chloro substituted; and
d) substitution of chloro at position 5 in the compound D obtained in step c) with an amine R₃NH₂ appropriately selected in order to obtain compounds of formula (I) with the desired R₃ group.

## Patentansprüche

1. Zusammensetzungen der allgemeine Formel (I) 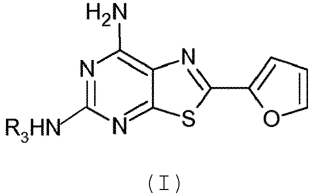 wobei R₃ ausgewählt ist aus der Gruppe, die besteht aus Wasserstoff, Alkyl optional substituiert, (CH₂)ₙ Aryl optional substituiert und (CH₂)ₙ Heteroaryl optional substituiert, wobei n eine ganze Zahl ist, die von 0 bis 4 reicht, und pharmazeutisch akzeptablen Salzen Tautomeren und Enantiomeren davon.

2. Zusammensetzungen nach Anspruch 1, wobei R₃ CH₂)ₙ Aryl oder (CH₂)ₙ Heteroaryl ist, optional substituiert, wobei n = 1 oder 2.

3. Zusammensetzungen nach einem der vorhergehenden Ansprüche, ausgewählt aus der Gruppe, die besteht aus:
2-(Furan-2-yl)-N⁵-(2-Metoxybenzyl)[1,3]Thiazolo[5,4-d]Pyrimidin-5,7-Diamin
2-(Furan-2-yl)-N⁵-(4-Metoxybenzyl)[1,3]Thiazolo[5,4-d]Pyrimidin-5,7-Diamin
2-(Furan-2-yl)-N⁵-(3-Metoxybenzyl)[1,3]Thiazolo[5,4-d]Pyrimidin-5,7-Diamin
3-(((7-Amino-2-(Furan-2-yl)[1,3]Thiazolo[5,4-d]Pyrimidin-5-yl)-Amino)Methyl)Phenol
2-(Furan-2-yl)-N⁵-(Furan-2-Ylmethyl)[1,3]Thiazolo[5,4-d]Pyrimidin-5,7-Diamin
2-(Furan-2-yl)-N⁵-(Thiophen-2-Ylmethyl)[1,3]Thiazolo[5,4-d]Pyrimidin-5,7-Diamin
2-(Furan-2-yl)-N⁵-(Pyridin-3-Ylmethyl)[1,3]Thiazolo[5,4-d]Pyrimidin-5,7-Diamin
2-(Furan-2-yl)-N⁵-(Pyridin-2-Ylmethyl)[1,3]Thiazolo[5,4-d]Pyrimidin-5,7-Diamin
N⁵-(3-Fluorobenzyl)-2-(Furan-2-yl)[1,3]Thiazolo[5,4-d]Pyrimidin-5,7-Diamin
2-(Furan-2-yl)-N⁵-(2-(Tiophen-2-yl)Ethyl)-[1,3]Thiazolo[5,4-d]Pyrimidin-5,7-Diamin
2-(Furan-2-yl)-N⁵-Propyl-[1,3]Thiazolo[5,4-d]Pyrimidin-5,7-Diamin
N⁵-Butyl-2-(Furan-2-yl)[1,3]Thiazolo[5,4-d]Pyrimidin-5,7-Diamin.

4. Pharmazeutische Zusammensetzung, die als den aktiven Grundbestandteil wenigstens eine Zusammensetzung der allgemeinen Formel (I) enthält, wie sie in den Ansprüchen 1 bis 3 definiert ist, unter Beigabe von einem oder mehreren Hilfsstoff(en) und/oder Verdünnung(en) und/oder pharmazeutisch akzeptablen Träger(n).

5. Pharmazeutische Zusammensetzung nach Anspruch 4, wobei ferner ein oder mehrere weitere(r) aktive(r) Grundbestandteile enthalten ist/sind.

6. Zusammensetzungen nach einem der Ansprüche 1 bis 3, zur Verwendung als Medikamente.

7. Zusammensetzungen nach einem der Ansprüche 1 bis 3, zur Verwendung bei der therapeutischen Behandlung von Erkrankungen oder Beschwerden, ansprechend auf die Blockade des Adenosin A_{2A} Rezeptors.

8. Zusammensetzungen zur Verwendung nach Anspruch 7, wobei die Erkrankungen oder Beschwerden ausgewählt sind aus der Gruppe, die besteht aus neurologischen Pathologien, Schmerz, Krebs, dermale Fibrose und Narbenbildung.

9. Verfahren zur Herstellung der Zusammensetzungen der allgemeinen Formel (I), wie sie in den Ansprüchen 1 bis 3 definiert ist, enthaltend die folgenden Schritte gemäß dem nachfolgend illustrierten Schema: 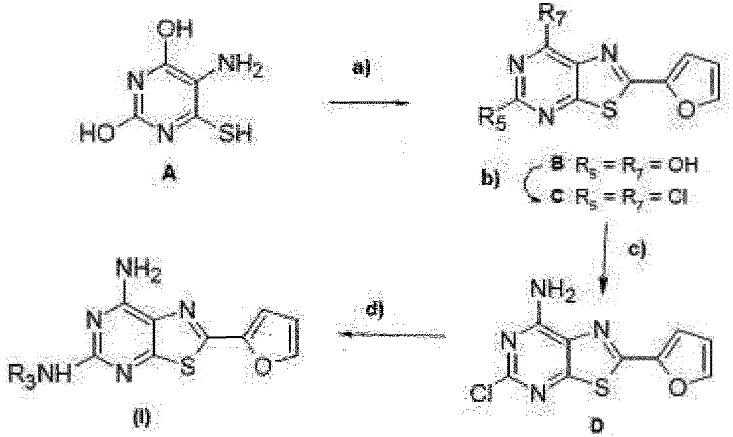 wobei R₃ wie in den Ansprüchen 1 bis 3 definiert ist:
a) Reaktion der Startzusammensetzung A, 5 Amino-6-Sulphanylpyrimidin-2,4-Diol, mit 2-Furoylchlorid, um eine Zusammensetzung B zu bilden, die das 2-(Furan-2-yl)-Thiazolo[5,4-d]Pyrimidin-5,7-Diol ist;
b) Chlorination der Zusammensetzung B, die im Schritt a) erhalten wurde, mit Substitution der zwei HydroxylGruppen und Bildung einer 5,7-Dichloro-Derivative C;
c) Substitution von Chlor an der Position 7 der Zusammensetzung C, die im Schritt b) erhalten wurde, durch ihr Reagieren mit einer wässrigen Lösung von Ammoniak um eine Zusammensetzung D 7-Amino-5-Chlor-Substituiert zu bilden; und
d) Substitution von Chlor an der Position 5 der Zusammensetzung D, die im Schritt c) erhalten wurde, mit einem Amin R₃NH₂, das geeignet ausgewählt ist, um Zusammensetzungen der Formel (I) mit der gewünschten R₃-Gruppe zu erhalten.

## Revendications

1. Composés de formule générale (I) 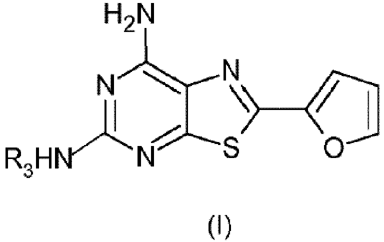 où R₃ est choisi dans le groupe suivant hydrogène, alkyle optionnellement substitué, (CH₂)ₙaryle optionnellement substitué et (CH₂)ₙhétéroaryle optionnellement substitué, où n est un nombre entier de 0 à 4,
et leurs sels, tautomères et énantiomères pharmaceutiquement acceptables.

2. Les composés selon la revendication 1, dans lesquels R₃ est (CH₂)ₙaryle ou (CH₂)ₙhétéroaryle, optionnellement substitué, où n = 1 ou 2.

3. Les composés selon l'une quelconque des revendications précédentes, choisis dans le groupe suivant :
2-(furane-2-yl)-N⁵-(2-métoxybenzyle)[1,3]thiazolo[5,4-d]pyrimidine-5,7-diamine
2-(furane-2-yl)-N⁵-(4-métoxybenzyle)[1,3]thiazolo[5,4-d]pyrimidine-5,7-diamine
2-(furane-2-yl)-N⁵-(3-métoxybenzyle)[1,3]thiazolo[5,4-d]pyrimidine-5,7-diamine
3-(((7-amino-2-(furane-2-yl)[1,3]thiazolo[5,4-d]pyrimidine-5-yl)amino)méthyle) phénol
2-(furane-2-yl)-N⁵-(furane-2-ylméthyle)[1,3]thiazolo [5,4-d]pyrimidine-5,7-diamine
2-(furane-2-yl)-N⁵-(thiophène-2-ylméthyle[1,3]thiazolo[5,4-d]pyrimidine-5,7-diamine
2-(furane-2-yl)-N⁵-(pyridine-3-ylméthyle)[1,3]thiazolo[5,4-d]pyrimidine-5,7-diamine
2-(furane-2-yl)-N⁵-(pyridine-2-ylméthyle)[1,3]thiazolo[5,4-d]pyrimidine-5,7-diamine
N⁵-(3-fluorobenzyl)-2-(furane-2-yl)[1,3]thiazolo[5,4-d]pyrimidine-5,7-diamine
2-(furane-2-yl)-N⁵-(2-(thiophène-2-yl)éthyle)-[1,3]thiazolo[5,4-d]pyrimidine-5,7-diamine
2-(furane-2-yl)-N⁵-propyle-[1,3]thiazolo[5,4-d]pyrimidine-5,7-diamine, et
N⁵-butyl-2-(furane-2-yl)[1,3]thiazolo[5,4-d]pyrimidine-5,7-diamine.

4. Une composition pharmaceutique comprenant comme principe actif au moins un composé de formule générale (I) tel que défini dans les revendications 1 à 3, en mélange avec un ou plusieurs excipients et/ou diluants et/ou supports pharmaceutiquement acceptables.

5. La composition pharmaceutique selon la revendication 4, comprenant en un ou plusieurs principes actifs supplémentaires.

6. Composés selon l'une quelconque des revendications 1 à 3, destinés à être utilisés comme médicaments.

7. Composés selon l'une quelconque des revendications 1 à 3, destinés à être utilisés dans le traitement thérapeutique de maladies ou de troubles :
répondant au blocage du récepteur A₂ₐ de l'adénosine.

8. Composés pour une utilisation selon la revendication 7, lorsque les maladies ou troubles appartiennent au groupe suivant : pathologies neurologiques, douleur, cancer, fibrose cutanée et cicatrices.

9. Un procédé pour la préparation des composés de formule générale (I) tels que définis dans les revendications 1 à 3, comprenant les étapes suivantes selon le schéma illustré ci-dessous : 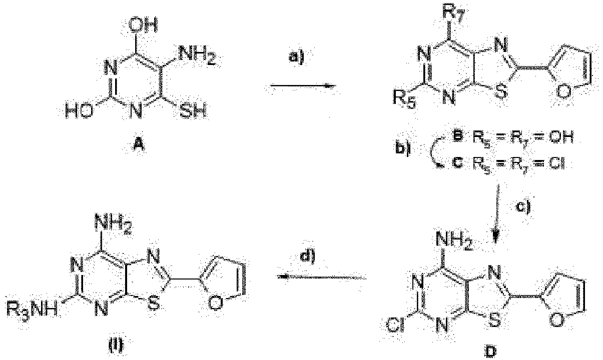 où R₃ est défini comme dans les revendications 1 à 3 :
a) réaction du composé de départ A, 5-amino-6-sulfanylpyrimidine-2,4-diol, avec du 2-furoyl chlorure pour former un composé B, qui est le 2-(furane-2-yl)-thiazolo [5,4-d] pyrimidine-5,7-diol ;
b) chloration du composé B obtenu à l'étape a) avec substitution des deux groupes hydroxyle et formation d'un dérivé C 5,7-dichloré ;
c) substitution du chlore en position 7 dans le composé C obtenu à l'étape b) en le faisant réagir avec une solution aqueuse d'ammoniaque, pour former un composé D 7-amino-5-chloré substitué ; et
d) substitution du chlore en position 5 dans le composé D obtenu à l'étape c) avec une amine R₃NH₂ choisie de manière appropriée pour obtenir des composés de formule (I) avec le groupe R₃ souhaité.
